# EUROPEAN PATENT APPLICATION

(11) **EP 3 139 338 A1**
(43) Date of publication of application: **08.03.2017**
(21) Application number: 15785267.4
(22) Date of filing: 27.04.2015
(51) Int. Cl.: G06Q 50/22, G06Q 10/10

(54) **SERVER FOR INFORMATION MANAGEMENT SUPPORT SYSTEM, CONTROL METHOD THEREFOR, AND CONTROL PROGRAM THEREFOR**

(30) Priority: 01.05.2014 JP 2014094671
(71) Applicant: Nagai, Chieko, Sakaide-shi, Kagawa 762-0025 (JP)
(72) Inventor: SUGIMOTO, Yukio, Tokyo 104-8280 (JP); TSUKAHARA, Shinnosuke, Tokyo 104-8280 (JP); OIWA, Takao, Muroran-shi Hokkaido 050-0084 (JP); NISHIMURA, Keita, Muroran-shi Hokkaido 050-0084 (JP); NAGAI, Chieko, Sakaide-shi, Kagawa 762-0025 (JP)
(74) Representative: Perrot, Emilie
(86) International application number: PCT/JP2015/062730
(87) International publication number: WO 2015/166925

(57) **Abstract**

The present invention provides a server and the like for an information management support system by which workload on a service provider is reduced, thereby enabling the efficiency of provision of services to be improved. A server according to one aspect of the present embodiment is equipped with: a registration part that stores received input text information as history information for form history information and history information for text history information that are stored in a storage unit; an extraction part that extracts, from the text history information stored in the storage unit, history information for at least one item in a form prepared for a service; and a form output part that causes an input-enabled area and/or a neighboring input-enabled area to be output together with form image information to a portable terminal of a service provider, said input-enabled area being a display area in which the extracted history information for the at least one item in the form prepared for the service can be viewed by the service provider.

## Description

### TECHNICAL FIELD

The present invention relates to a server in an information management support system, a control method thereof, and a control program thereof.

### BACKGROUND ART

A service provided by a nursing care center, such as a medical institution or a welfare institution manages information by using a number of paper forms for services. For example, in the home nursing care service, a large number of types of forms are used for one patient, such as a home nursing care plan which is information on a plan, a home service record which is a record at the time of actual visit, home nursing care instructions from a doctor, and a form indicating basic information on a patient other than the above. The required forms increase in number each time a service provider visits a patient.

From the viewpoint of security, it is unfavorable for a service provider to visit a plurality of patients while carrying the necessary forms, in which personal information is described. Thus, when a service provider visits a plurality of patients in a day, the service provider usually returns to the home service center after visiting a patient, and replaces the forms for the patient with another form for the next patient. The service provider spends a lot of time during round trips between visiting patients and the home service center, which results in a lot of time traveling.

On the other hand, these paper forms are currently being computerized. For example, according to the method disclosed in Patent Document 1, all of the information may be managed by a computer and be browsed and shared on the internet. If a service provider carries a mobile device, the service provider may browse and input all of the information while visiting the patient, and therefore efficiency is improved compared to the conventional information management by paper media.

### Related Document

Patent Document 1 Japanese Laid Open Patent No. 2002-215800

Further, in order to use a conventional paper form, the paper form is scanned, character data from the scanned form image is read by an OCR (Optical Character Reader), and information that is input on the input screen is outputted onto the internet on a browsing screen of an electronic form having the same layout as the paper form.

### SUMMARY OF THE INVENTION

However, the layout and the screen are often changed from those of the conventional paper forms so as to be suitable for electronic browsing, which will require a lot of trainings for a service provider who is familiar with the layout of the conventional paper form so as to be familiar with browsing and sharing of information on the internet.

On the other hand, if character data is read by an OCR from a form image obtained by scanning a paper form, in order to browse the character data later, it is also necessary to read the form image in addition to the read character data in order for the read character data, and therefore it takes a much time until the form image including the character data is read.

Further, even if information is output on a browsing screen of an electronic form having the same layout as that of the paper form, the input screen on the web is different from the browsing screen, and therefore it is not easy for a service provider to use the input screen.

In other words, although the conventional form image method or the electronic form method scans a paper form and extracts information, such as character data and/or layout, the use efficiency of the information is not sufficient. These problems are not limited to the above-described home nursing care service and similarly occur in various fields, such as in computerization of a paper medical chart or the like within a medical institution, in prescription in a home medication service, and in learning history information management of a student attending at a lecture in a home teacher service.

The present invention has been provided in view of these circumstances, and an object of the present invention is to provide a server in an information management support system which reduces the work load for a service provider and to improve service provision efficiency when a service provider company provides services, a control method of the server, and a control program of the server.

A control method of a server according to an aspect of the present embodiment is a control method of a server in an information management support system comprising a service provider side mobile device relating to a service provider who provides a service and a server capable of communicating with the service provider side mobile device, the server comprising a storage unit configured to store: form history information including form image information on a form that is generated for a service provided by a service provider; and history information input in correspondence to at least one item of the form image information and written history information indicating written information of history information input in correspondence to at least one item of a form that is generated for the service for each patient, and the server performs:
a step of receiving written information input for a form that is generated for a service provided by a service provider from the service provider side mobile device;
a step of storing the received input written information as history information of the form history information stored in the storage unit and as history information of the written history information stored in the storage unit;
a step of extracting history information on at least one item of a form that is generated for a service from the written history information stored in the storage unit; and
a step of causing the service provider side mobile device to output, along with form image information, an input possible area and/or an area in the vicinity of the input possible area displaying the extracted history information on at least one item of a form that is generated for a service so that a service provider can browse the history information.

It is preferable for the control method of a server according to an aspect of the present embodiment to store, in the storing step, an execution date and/or an execution time as history information of the form history information stored in the storage unit and the written history information stored in the storage unit, to further perform a step of receiving a past date and/or time input by a service provider from the service provider side mobile device, and to extract, in the extracting step, history information on at least one item of a form that is generated for a service, which is specified by the received past date and/or time, from the written history information stored in the storage unit.

It is preferable for the control method of a server according to an aspect of the present embodiment to further store execution plan information indicating a plan of performing a service of at least one item of a form that is generated for a service provided by a service provider in the storage unit, to extract execution plan information stored in the storage unit indicating a plan of performing a service of at least one item of a form that is generated for a service in the extracting step, and to output a display area displaying the extracted execution plan information indicating a plan of performing a service of at least one item of a form that is generated for a service and an input possible area into which an execution record is input by a service provider who performs the service along with form image information in the outputting step.

It is preferable for the control method of a server according to an aspect of the present embodiment to further store form layer information including form image information on a form that is generated for a service and arrangement information on an input possible area and/or a display area in the storage unit, to further perform a step of acquiring form image information obtained by scanning a form that is generated for a service, a step of acquiring arrangement information defining an input possible area and/or a display area in the acquired form image information, and a step of storing the acquired form image information and the acquired arrangement information on an input possible area and/or a display area in the form layer information stored in the storage unit on a form that is generated for a service, and to arrange an input possible area and/or a display area in form image information based on arrangement information of the form layer information stored in the storage unit on a form that is generated for a service and further, to display the extracted history information on at least one item of a form that is generated for a service in an input possible area and/or a display area arranged in the form image information so that a service provider can browse the history information in the outputting step.

A server according to an aspect of the present embodiment is a server in an information management support system comprising a service provider side mobile device relating to a service provider who provides a service and a server capable of communicating with the service provider side mobile device, the server comprising:
a storage unit configured to store: form history information including form image information on a form that is generated for a service provided by a service provider and history information input in correspondence to at least one item of the form image information; and written history information indicating written information of history information input in correspondence to at least one item of a form that is generated for the service for each patient:
   a communication unit configured to receive written information input for a form that is generated for a service provided by a service provider from the service provider side mobile device;
   a registration unit configured to store the received input written information as history information of the form history information stored in the storage unit and as history information of the written history information;
   an extraction unit configured to extract history information on at least one item of a form that is generated for a service from the written history information stored in the storage unit; and
   a form output unit configured to cause the service provider side mobile device to output, along with form image information, an input possible area and/or an area in the vicinity of the input possible area displaying the extracted history information on at least one item of a form that is generated for a service so that a service provider can browse the history information.

A control program of a server according to an aspect of the present embodiment is a control program of a server in an information management support system comprising a service provider side mobile device relating to a service provider who provides a service and a server capable of communicating with the service provider side mobile device, the server comprising a storage unit configured to store: form history information including form image information on a form that is generated for a service provided by a service provider and history information input in correspondence to at least one item of the form image information; and written history information indicating written information of history information input in correspondence to at least one item of a form that is generated for the service for each patient, and the control program causes the server to perform:
a step of receiving written information input for a form that is generated for a service provided by a service provider from the service provider side mobile device;
a step of storing the received input written information as history information of the form history information stored in the storage unit and as history information of the written history information stored in the storage unit;
a step of extracting history information on at least one item of a form that is generated for a service from the written history information stored in the storage unit; and
a step of causing the service provider side mobile device to output, along with form image information, an input possible area and/or an area in the vicinity of the input possible area displaying the extracted history information on at least one item of a form that is generated for a service so that a service provider can browse the history information.

The server, the control method thereof, and the control program thereof according to an aspect of the present embodiment store written information input the last time or in the past also as written history information separately from form history information and extract the corresponding written information input the last time or in the past for a service provider from the written history information, not from the form history information. The server, the control method thereof, and the control program thereof according to an aspect of the present embodiment can narrow down the range of the extraction target by removing the form image information from the range of the extraction target, and therefore a patient can quickly extract the corresponding written information input the last time or in the past.

Further, the server, the control method thereof, and the control program thereof according to an aspect of the present embodiment display the corresponding written information input the last time or in the past for a patient in the input possible area and/or the vicinity thereof, and therefore a service provider may easily grasp a patient's condition change between the last or a previous time and this time.

Furthermore, a service provider can fill in a form based on or by referring to the written information input the last time or in the past, which is displayed in the input possible area, and therefore the server, the control method thereof, and the control program thereof according to an aspect of the present embodiment can reduce the work load in order to fill in a form.

Still furthermore, the server, the control method thereof, and the control program thereof according to an aspect of the present embodiment output the input possible area and/or the area in the vicinity of the input possible area along with the form image information, and therefore a service provider can browse and fill in a paper form without a feeling of difference within the same screen as the screen obtained by outputting the form image information.

### BRIEF DESCRIPTION OF THE DRAWINGS

FIG. 1 is a diagram illustrating an example of an outline configuration of an information management support system;
FIG. 2 is a diagram illustrating an example of an outline configuration of a service provider side mobile device;
FIG. 3 is a diagram illustrating an example of an outline configuration of a server;
FIG. 4A is a diagram illustrating an example of a data structure of one of various management tables;
FIG. 4B is a diagram illustrating another example of a data structure of one of various management tables;
FIG. 4C is a diagram illustrating still another;
   example of a data structure of one of various management tables;
FIG. 5A is a diagram illustrating still another example of a data structure of one of various management tables;
FIG. 5B is a diagram illustrating still another example of a data structure of one of various management tables;
FIG. 6A is a diagram illustrating an example of an outline configuration when displaying extracted form layer information and history information;
FIG. 6B is a diagram illustrating an example of an outline configuration when displaying the extracted form layer information and history information;
FIG. 7 is a diagram illustrating an example of an operation flow of the server;
FIG. 8 is a diagram illustrating an example of scanned form image information;
FIG. 9 is a diagram illustrating an example of form image information in which arrangement information on an input possible area and/or a display area is defined;
FIG. 10 is a diagram illustrating an example of an operation sequence by the information management support system;
FIG. 11 is a diagram illustrating an example of output form information;
FIG. 12 is a diagram illustrating an example of form information into which written information is input.
FIG. 13 is a diagram illustrating another example of form image information in which arrangement information on an input possible area and/or a display area is defined;
FIG. 14 is a diagram illustrating another example of an operation sequence by the information management support system;
FIG. 15 is a diagram illustrating another example of output form information;
FIG. 16 is a diagram illustrating a selection input of another example of output form information; and
FIG. 17 is a diagram illustrating another example of output form information.

### EMBODIMENTS FOR EMBODYING THE PRESENT INVENTION

Hereinafter, with reference to the drawings, various embodiments of the present invention are explained. However, it should be noted that the technical scope of the present invention is not limited to those embodiments, but encompasses the inventions described in the claims and equivalents thereof.

First, an outline of the present embodiment is explained below.

In the present embodiment, a service provider side mobile device 2 requests form information in which the corresponding written history information of the last time (or a past date) is displayed for a patient from a server 3. The server 3 transmits the form information displaying the corresponding written history information of the last time (or a past data) for a patient requested by the service provider side mobile device 2 to the service provider side mobile device 2. The service provider side mobile device 2 outputs the form information received from the receiver 3 and displaying the corresponding written history information of the last time (or a past date) for a patient.

A service provider provides a service to a patient while appropriately browsing the form information output to the service provider side mobile device 2. Then, the service provider inputs, via an operation unit 23, written information about a record of the service provided to the patient in an input possible area of the form information output to the service provider side mobile device 2.

The service provider side mobile device 2 transmits the written information input via the operation unit 23 to the server 3. The server 3 stores the input written information received from the service provider side mobile device 2 as history information of the form history information and history information of the written history information. Although one example where the input information is written information is explained, the input information is not limited to written information and may include, for example, image information, voice information, motion picture information, etc.

Further, in the information management support system in the present embodiment, a service provided by a service provider is a home nursing care service, and managed information is information relating to a home service. However, the present invention is not limited to the embodiment. The service may be a home nursing care service provider, a homemaker service provider, a babysitter service provider, a home teacher service provider, or nursing care provider, diagnosis, rehabilitation, medical examination, dispensing, dental examination, etc., in an institution, such as a hospital, or any other service which the present invention can be applied.

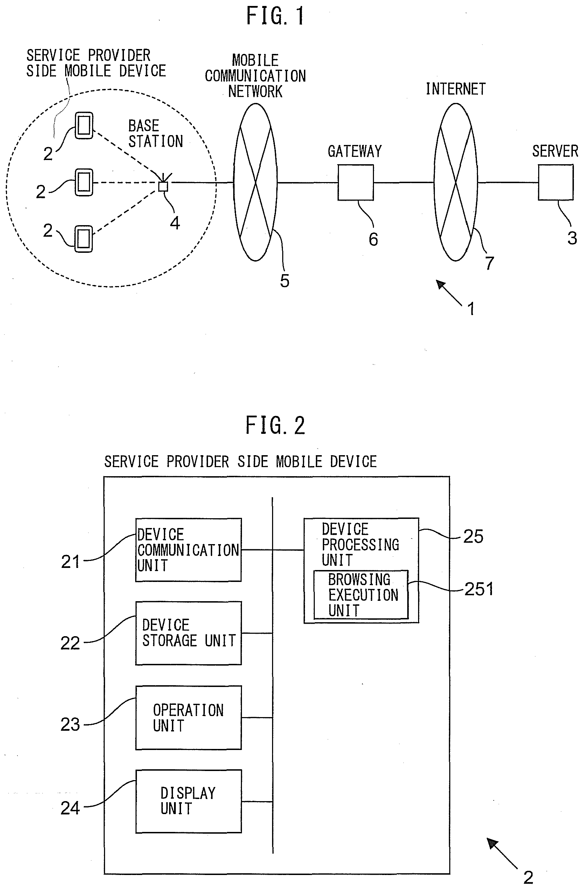

FIG. 1 is a diagram illustrating an example of an outline configuration of an information management support system 1.

The information management support system 1 includes at least the one service provider side mobile device 2 and the server 3. The service provider side mobile device 2 and the server 3 are interconnected via a communication network and for example, are interconnected via a base station 4, a mobile communication network 5, a gateway 6, and Internet 7. Programs (e.g., browsing program) executed by the service provider side mobile device 2 and programs (e.g., information management support program) executed by the server 3 are communicated by using a communication protocol, such as the hyper-text transfer protocol (HTTP).

FIG. 2 is a diagram illustrating an example of an outline configuration of the service provider side mobile device 2.

The service provider side mobile device 2 connects to the server 3 via the base station 4, the mobile communication network 5, the gateway 6, and the Internet 7 and communicates with the server 3. The service provider side mobile device 2 requests form information or the like displaying the corresponding written history information of the last time (or a past date) for a patient from the server 3 in accordance with an operation of the operation unit 23 (button or the like) by a service provider. Further, the service provider side mobile device 2 receives the form information displaying the corresponding written history information of the last time (or a past date) for a patient from the server 3 and outputs the form information. The service provider side mobile device 2 includes a device communication unit 21, a device storage unit 22, the operation unit 23, a display unit 24, and a device processing unit 25.

In the present embodiment, as the service provider side mobile device 2, a tablet device is supposed, but the present invention is not limited to this. The service provider side mobile device 2 may be a tablet PC, a note PC, a multifunction mobile telephone (so-called "smartphone"), a mobile telephone (so-called "feature phone"), a personal digital assistant (PDA), a wearable device, or any other device which the present invention can be applied to the device.

The device communication unit 21 includes a communication interface circuit including an antenna having a predetermined frequency band as a reception band and connects the service provider side mobile device 2 to a radio communication network. The device communication unit 21 establishes a radio signal line with the base station 4 by the CDMA (Code Division Multiple Access) scheme, the LTE (Long Term Evolution) scheme, etc., via a channel allocated by the base station 4 and communicates with the base station 4. Then, the device communication unit 21 transmits data supplied from the device processing unit 25 to the server 3 or the like. The device communication unit 21 supplies the data received from the server 3 or the like to the device processing unit 25.

The device storage unit 22 includes, for example, at least one of a semiconductor memory device, a magnetic disk device, and an optical disk device. The device storage unit 22 stores an operating system program, driver programs, application programs, data, etc., used for processing in the device processing unit 25. For example, the device storage unit 22 stores an input device driver program that controls the operation unit 23, an output device driver program that controls the display unit 24, etc., as the driver programs. Further, the device storage unit 22 stores a browsing program or the like that acquires and outputs form information displaying the written history information of the last time (or a past date) as the application program. Furthermore, the device storage unit 22 stores form information or the like displaying the written history information of the last time (or a past date) as the data. In addition to the above, the device storage unit 22 may temporarily store temporal data according to predetermined processing.

The operation unit 23 may be a touch panel, a touch pad, a keyboard, or any device which the device can perform the operation of the service provider side mobile device 2. A service provider may input characters, figures, etc., by using the operation unit 23. Further, a service provider can input by hand writing by using the operation unit 23. Furthermore, although not illustrated schematically, a service provider can input data by selecting a file of data of an image photographed by a camera, motion picture data, and data of voice recorded by a microphone by using the operation unit 23. When operated by a service provider, the operation unit 23 generates a signal corresponding to the operation. Then, the generated signal is supplied to the device processing unit 25 as the instructions of the service provider.

The display unit 24 may also be a liquid crystal display, an organic EL (ElectroLuminescence) display, or any device which the device can output video, image, character, etc. The display unit produces a display in accordance with data supplied from the device processing unit 25.

The device processing unit 25 includes one or more processors and peripheral circuits thereof. The device processing unit 25 centrally controls the entire operation of the service provider side mobile device 2 and for example, is a CPU (Central Processing Unit). The device processing unit 25 controls the operations of the device communication unit 21, the display unit 24, etc., so that various kinds of processing of the service provider side mobile device 2 are performed in an appropriate procedure in accordance with the programs, the operation of the operation unit 23, etc. The device processing unit 25 performs processing based on the programs (operating system program, driver program, application program, etc.) stored in the device storage unit 22. Further, the device processing unit 25 can parallely execute a plurality of programs (application programs or the like).

The device processing unit 25 includes at least a browsing execution unit 251. Each unit is a function module that is implemented by a program executed by a processor included in the device processing unit 25. Alternatively, each unit may be packaged on the service provider side mobile device 2 as firmware.

The browsing execution unit 251 acquires and displays display data relating to creation of a service provision plan or the like. In other words, the browsing execution unit 251 transmits a request for form information displaying the corresponding written history information of the last time (or a past date) for a patient to the server 3 via the device communication unit 21 in accordance with instructions from a service provider. Further, the browsing execution unit 251 receives the corresponding form information from the server 3 via the device communication unit 21. The browsing execution unit 251 generates drawing data based on the received form information. in accordance with instructions from a service provider, the browsing execution unit 251 analyzes the received form information and specifies the control data and the contents data, and lays out the contents data similarly specified in accordance with the specified control data, and generates drawing data. Then, the browsing execution unit 251 outputs the generated drawing data to the display unit 24. By the browsing execution unit 251, the generated drawing data is output to and displayed on the display unit 24, and characters or the like are input into an input possible area of the information displayed on the display unit 24 by the operation unit 23.

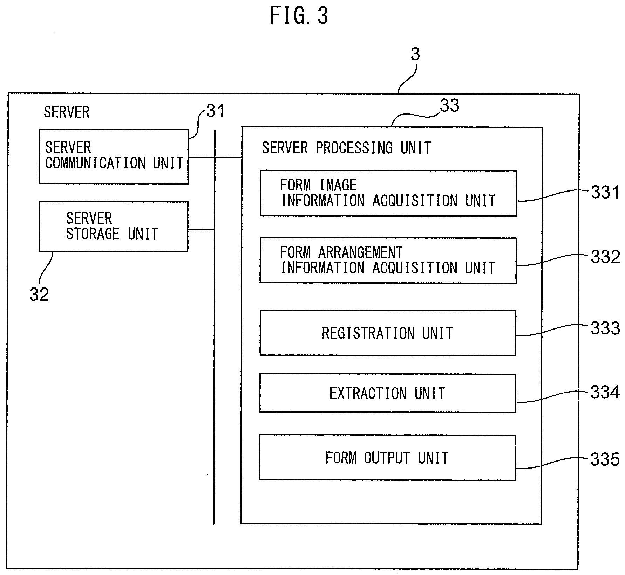

FIG. 3 is a diagram illustrating an example of an outline configuration of the server 3.

The server 3, for example, outputs form information in accordance with a request from the service provider side mobile device 2. Further, the server 3 transmits the output form information to the service provider side mobile device 2. The server 3 includes a server communication unit 31, a server storage unit 32, and a server processing unit 33.

The server communication unit 31 includes a communication interface circuit for connecting the server 3 to the Internet 7 and communicates with the Internet 7. Then, the server communication unit 31 supplies the data received from the service provider side mobile device 2 or the like to the server processing unit 33. Further, the server communication unit 31 transmits the data supplied from the server processing unit 33 to the service provider side mobile device 2 or the like.

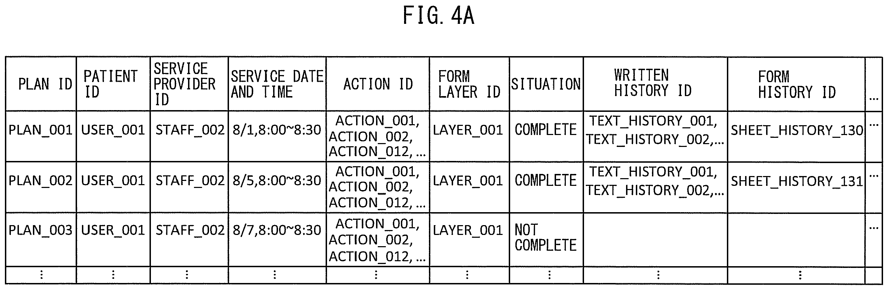

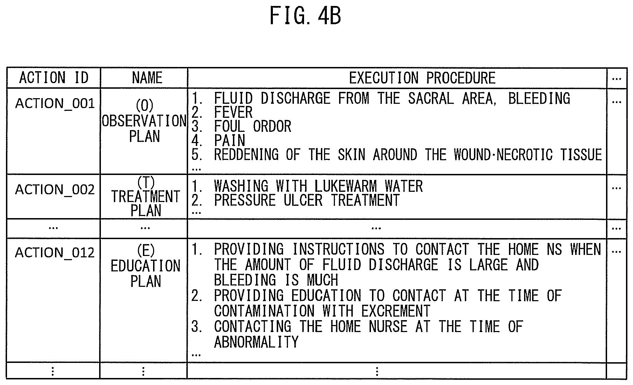

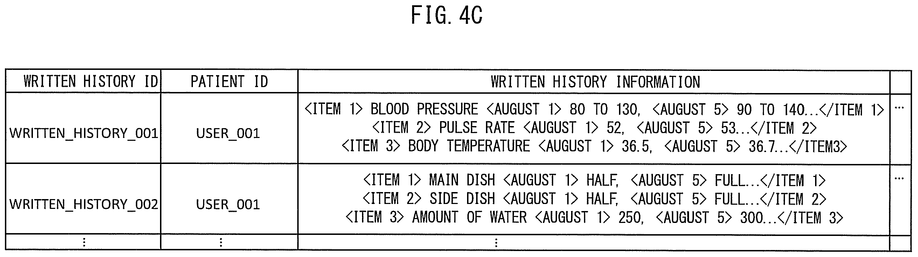

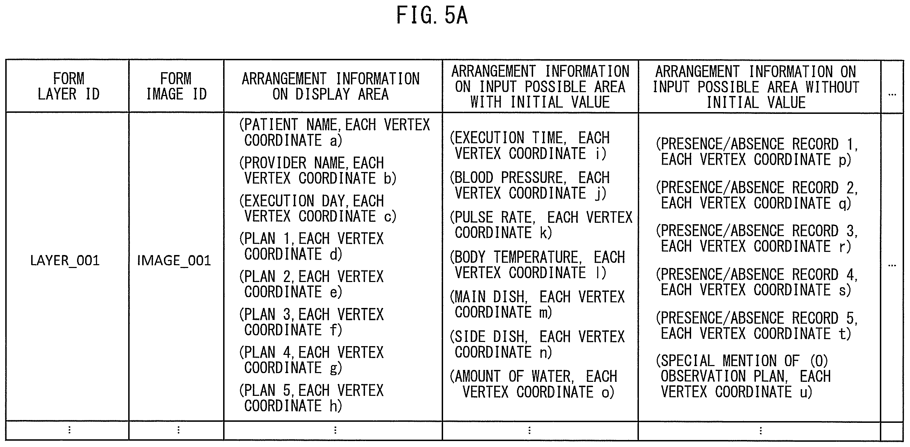

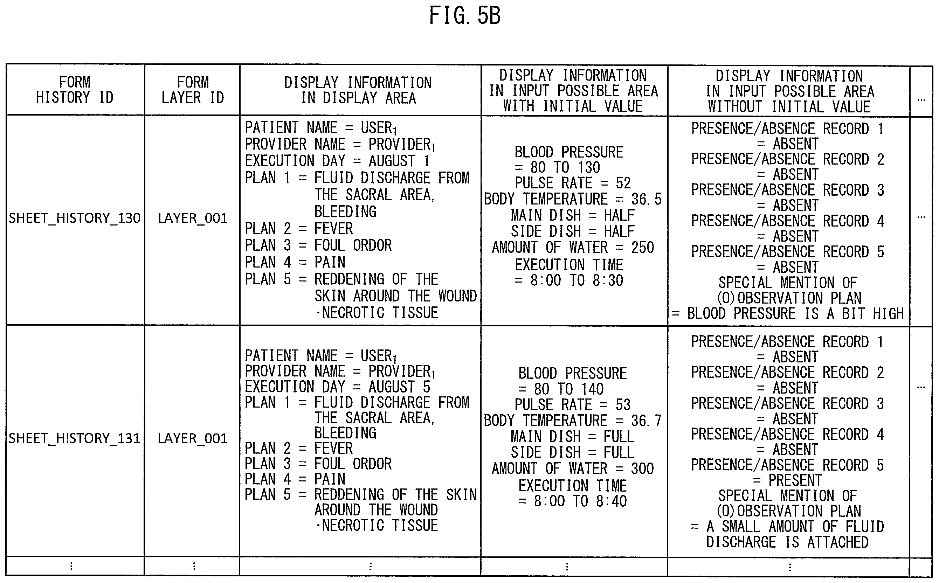

The server storage unit 32 includes, for example, at least one of a magnetic tape device, a magnetic disk device, a flash memory, an optical disk device, and another memory. The server storage unit 32 stores an operating system program, driver programs, application programs, data, etc., used for processing in the server processing unit 33. For example, the server storage unit 32 stores, as the application program, an information management support program or the like that causes form information displaying the corresponding written history information of the last time (or a past date) for a patient to be output. Further, the server storage unit 32 stores, as the data, a service provision plan management data (FIG. 4A) for managing a service provision plan, an execution procedure management table (FIG. 4B) for managing an execution procedure of a service action, a written history information management table (FIG. 4C) for managing written history information, a form layer information management table (FIG. 5A) for managing form layer information, a form history information management table (FIG. 5B) for managing form history information, etc. Furthermore, the server storage unit 32 may temporarily store temporary data relating to predetermined processing.

The server processing unit 33 includes one or a plurality processors and peripheral circuits thereof. The server processing unit 33 centrally controls the entire operation of the server 3 and for example, is a CPU. The server processing unit 33 controls the operation of the server communication unit 31 or the like so that the various kinds of processing of the server 3 are performed in an appropriate procedure in accordance with the programs stored in the server storage unit 32, requests from the service provider side mobile device 2, etc. The server processing unit 33 performs processing based on the programs (operating system program, driver program, application program, etc.) stored in the server storage unit 32. Further, the server processing unit 33 can parallelly execute a plurality of programs (application programs or the like).

The server processing unit 33 includes a form image information acquisition unit 331, a form arrangement information acquisition unit 332, a registration unit 333, an extraction unit 334, and a form output unit 335. Each of these units is a function module that is implemented by a program executed by a processor included in the server processing unit 33. Alternatively, each of these units may be packaged on the server 3 as firmware.

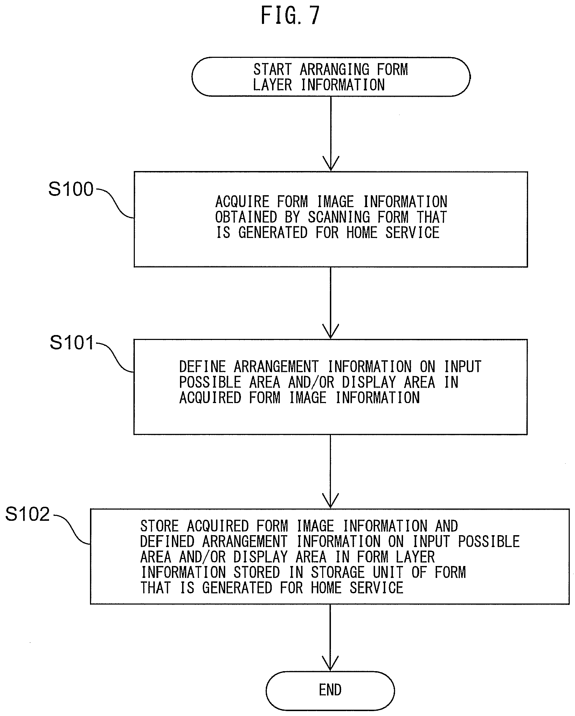

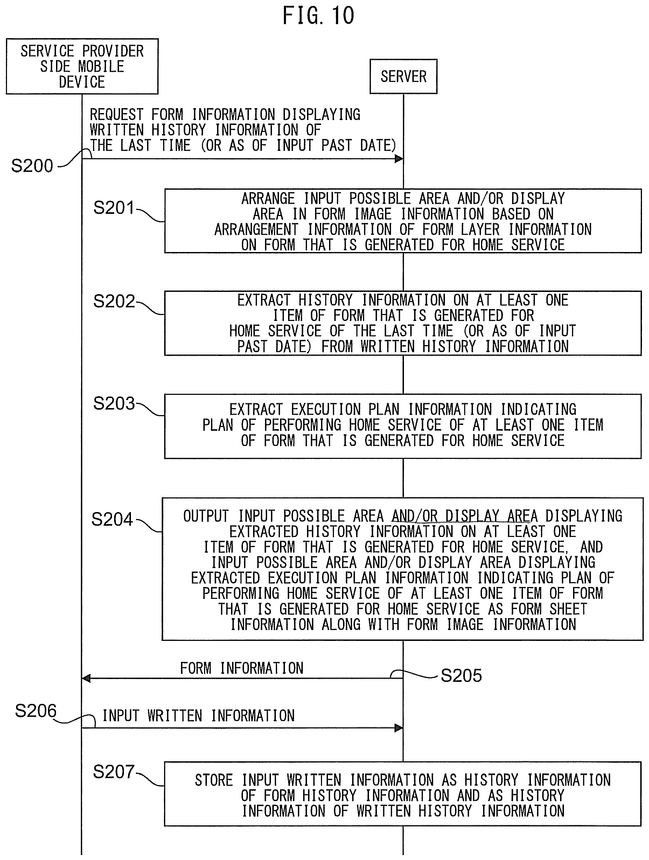

The form image information acquisition unit 331 performs step S100 (see FIG. 7) of an arrangement operation flow of form layer information by the server. The form arrangement information acquisition unit 332 performs step S101 (see FIG. 7) of the arrangement operation flow of the form layer information by the server. The registration unit 333 performs step S102 (see FIG. 7) of the arrangement operation flow of the form layer information by the server and step S207 (see FIG. 10) of an operation sequence by the information management support system 1.

The extraction unit 334 performs steps S202 and S203 (see FIG. 10) of the operation sequence by the information management support system 1. The form output unit 335 performs steps S201, S204, and S205 (see FIG. 10) of the operation sequence by the information management support system 1.

FIGs. 4A, 4B, and 4C and FIGs. 5A and 5B are diagrams illustrating examples of data structures of various management tables.

FIG. 4A illustrates a service provision plan management table for managing a service provision plan. As explained in the following, the service provision plan management table stores not only a future plan created in advance by a service provider but also the results of performing the plan. The service provision plan management table stores, for each individual service provision plan, the ID of the individual service provision plan, the ID of a patient relating to the individual service provision plan, the ID of a service provider, the date and time of the service provision, the ID of a provided service action, the ID of a form layer, the service provision situation ("not complete" or "completed"), the ID of a written history, the ID of a form history, etc. For convenience, as Patient ID, only "USER_001" is displayed, but information about another patient can be similarly managed in this service provision plan management table. The service provision situation is changed from "not complete" to "completed" when the form history information and the written history information are stored after the planned home service is performed.

The above-described information mainly relates to patients and schedules of service providers, but the present invention is not limited to this. Other than the information described above, information about various plans, such as the flow of the total work, may be included.

As described above, for a service to be provided to each patient, a plan created in advance by a service provider is registered in the service provision plan management table. Then, after the service provider provides the service based on the created plan, in the service provision plan management table, the history information on the results of performing the plan (form history information, written history information, etc.) is further accumulated.

FIG. 4B illustrates an execution procedure management table for managing an execution procedure of a service action. The execution procedure management table stores the ID, the name, the execution procedure, etc., of a service action for each service action. For example, in the ID "ACTION_001" of the service action to be provided, as the action of observation, the order of observation, such as "1. fluid discharge from the sacral area, bleeding", "2. fever", etc., is set in the execution procedure management table. The ID "ACTION_001" of the service action to be provided is associated with the ID of the service action to be provided stored in the column of Action ID of the service provision plan management table in FIG. 4A described above.

FIG. 4C illustrates a written history information management table for managing written history information. The written history information management table stores, for each written history, the ID of the written history, the ID of a patient, written history information, etc. The written history information represents the item of the written history information and its value by structured written, such as XML, for each execution date and/or execution time, and therefore it is easy to extract the item of the written history information. The written history information may be in any format, such as a CSV format, a JSON format, and a table format, as long as the item and its value are represented for each execution date and/or execution time. Further, the written history information of the written history information management table in FIG. 4C stores the written history information for each patient, but the written history information for each patient and service action can be integrally stored. The ID of the written history (e.g., "WRITTEN_HISTORY_001") is associated with the ID of the written history stored in the column of Written history ID of the service provision plan management table in FIG. 4A described above.

FIG. 5A illustrates a form layer information management table for managing form layer information. The form layer information management table stores, for each piece of form layer information, the ID of the form layer information, the ID of form image information, arrangement information on a display area, arrangement information on an input possible area with initial value, arrangement information on an input possible area without initial value, etc.

The display area is an area in which information that cannot be modified is displayed or an area in which displayed information can be selected. The input possible area is an area in which information that can be modified is displayed. The arrangement information on the display area, the arrangement information on the input possible area with initial value, and the arrangement information on the input possible area without initial value each indicate layer information, which is a pair of the item name of each area and each vertex coordinate of each area. Further, each vertex coordinate of each area may be held as a set with the item name of the area within the form history information, and only the item name of the area may be held as the form layer information.

The input possible area includes two kinds of input possible area. A first input possible area is an input possible area with initial value in which any item name is defined. In the input possible area with initial value, written history information is displayed as an initial value at the time of output of form information in accordance with the defined item name. A second input possible area is an input possible area without initial value for outputing form information.

Further, the input possible area without initial value may be a radio button without initial value. For example, a radio button without initial value, such as "∘absent ∘present", may be displayed in the input possible area without initial value with an item name as "presence/absence record". Further, a radio button without initial value, such as "∘ one ∘not complete", may be displayed in the input possible area without initial value with an item name as "completed/not complete record". The display in the input possible area without initial value may be a checkbox or the like, in place of a radio button and an item of any contents may be displayed. The ID of the form layer (e.g., "LAYER_001") is associated with the form layer ID stored in the column of Form layer ID of the service provision plan management table in FIG. 4A described above.

FIG. 5B illustrates a form history information management table for managing form history information. The form history information management table stores, for each form history, the ID of the form history, the ID of a form layer, display information in a display area, display information in an input possible area with initial value, display information in an input possible area without initial value, etc. The form history information management table is a table that is referred to when all the past form information, not only the past history information, including the form image information is requested.

The display information in the display area, the display information in the input possible area with initial value, and the display information in the input possible area without initial value each indicate a pair of the item name of each area and the written information input and displayed in each area. The ID of the form history (e.g., "SHEET_HISTORY_130") is associated with the form history ID stored in the column of Form history ID of the service provision plan management table in FIG. 4A described above.

As described above, the information registered in each table is associated with the ID and each table is designed so that the information associated with each other can be extracted by searching for information registered in each table with Patient ID by the extraction unit 334 of the server 3.

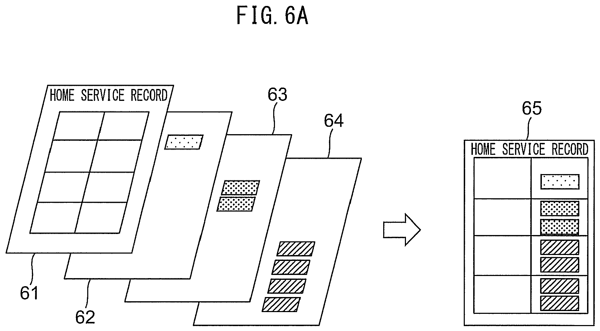

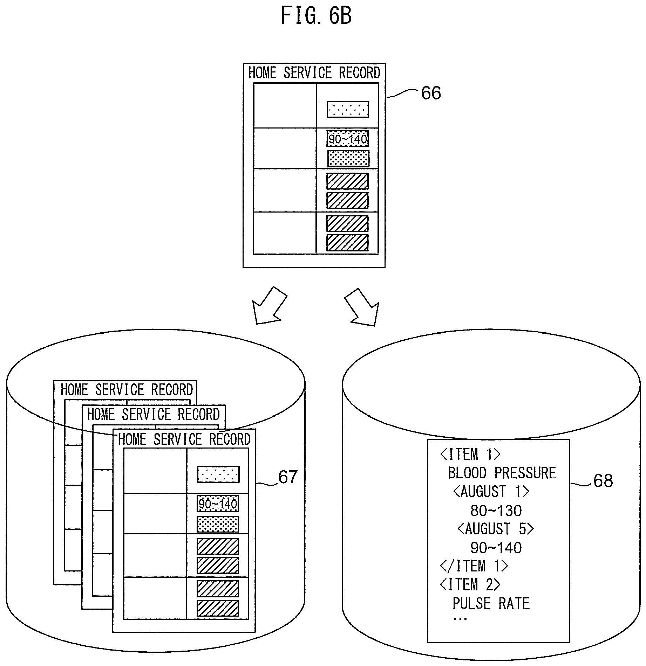

FIGs. 6A and 6B are diagrams illustrating examples of an outline configuration at the time of displaying extracted form layer information and history information.

On the left side of an arrow in FIG. 6A, form image information 61 obtained by scanning a paper form of a home service record, layer information 62 on a display area, layer information 63 on an input possible area with initial value, and layer information 64 on an input possible area without initial value are illustrated.

However, the form image information 61 does not necessarily need to be a form obtained by scanning a paper. For example, when a form is introduced newly, a form file generated electronically by word processor software, spread sheet calculation software, etc., may be used as data of the form image information 61 as it is.

On the right side of the arrow in FIG. 6A, form layer information 65 obtained by arranging in an overlap manner the layer information 62 on the display area, the layer information 63 on the input possible area with initial value displaying written history information as an initial value, and the layer information 64 on the input possible area without initial value on the form image information 61 obtained by scanning a paper form home service record and outputting the form image information 61 is illustrated. The form layer information 65 on the right side of the arrow in FIG. 6A seems one form, but it has a format configured by layers as illustrated on the left side of the arrow in FIG. 6A and the way the data is treated is different for different layers. Details will be described later.

Above arrows in FIG. 6B, form information 66 is illustrated, into the input possible area of which, written information "90 to 140" is input via the operation unit 23 of the service provider side mobile device 2 of a service provider.

Under the arrows in FIG. 6B, form history information 67 and written history information 68 that store the written information "90 to 140" input into the form information 66 in history information are illustrated. Thus, the extraction unit 334 can extract the corresponding written information input the last time or in the past for a patient from the written history information 68, not from the form history information 67. For example, when searching for past information, the extraction unit 334 can narrow down the range of the extraction target by removing the form image information 61 from the range of the extraction target, and therefore a patient can quickly extract the corresponding written information input the last time or in the past.

FIG. 7 is a diagram illustrating an example of an arrangement operation flow of form layer information by the server. With reference to FIG. 3, FIG. 8, and FIG. 9, the flow in FIG. 7 is explained.

The operation flow explained in the following is performed mainly by the server processing unit 33 in cooperation with each element of the server 3 based on the program stored in advance in the server storage unit 32.

The form image information acquisition unit 331 acquires the form image information 61 obtained by scanning a form that is generated for a home service (step S100). The process of step S100 is performed by scanning a form by a scanner, not illustrated in the server 3 in FIG. 3, or receiving the scanned form image information 61 via the server communication unit 31. The scanned form image information 61 may be image information in any format as long as it is image information on a form, such as PDF, JPG, GIF, and PNG, and further, the scanned form image information 61 may be form data electronically generated from the beginning by word processor software, spread sheet software, etc., as described above.

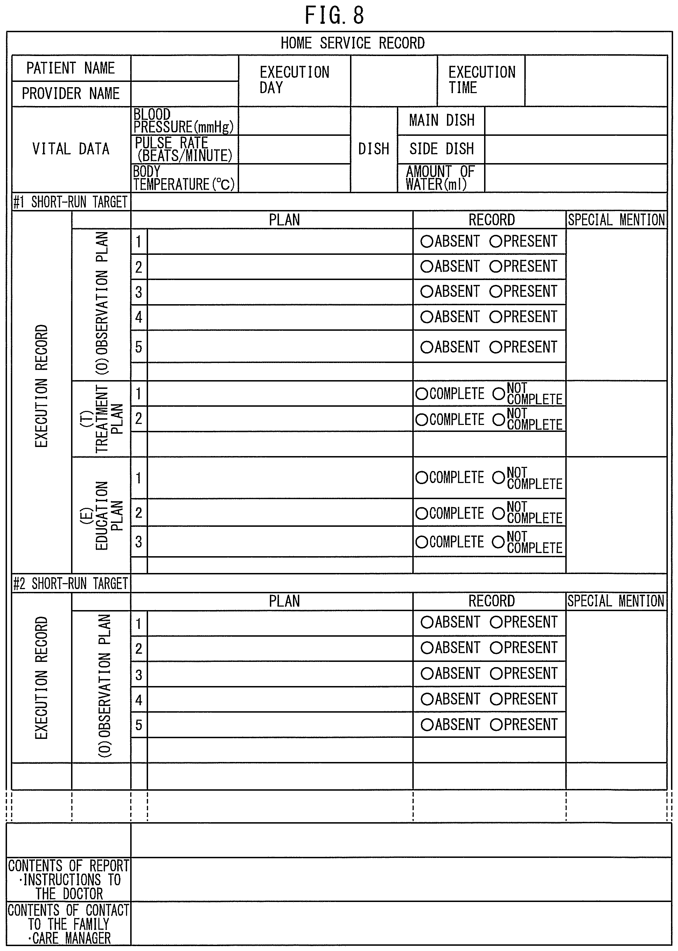

FIG. 8 is a diagram illustrating an example of the form image information 61 obtained by scanning a paper home service record. The scanned form image information 61 corresponds to a so-called original sheet, and as illustrated in FIG. 8, the scanned form image information 61 is a form in which every entry column is not filled in. The form image information 61 is generated by scanning a paper form used conventionally. Thus, a service provider uses the layout of a form with which the service provider is familiar conventionally, and therefore a service provider can input or brow effectively.

The form arrangement information acquisition unit 332 acquires arrangement information on the input possible area and/or the display area defined by a form creator for the acquired form image information 61 (step S101).

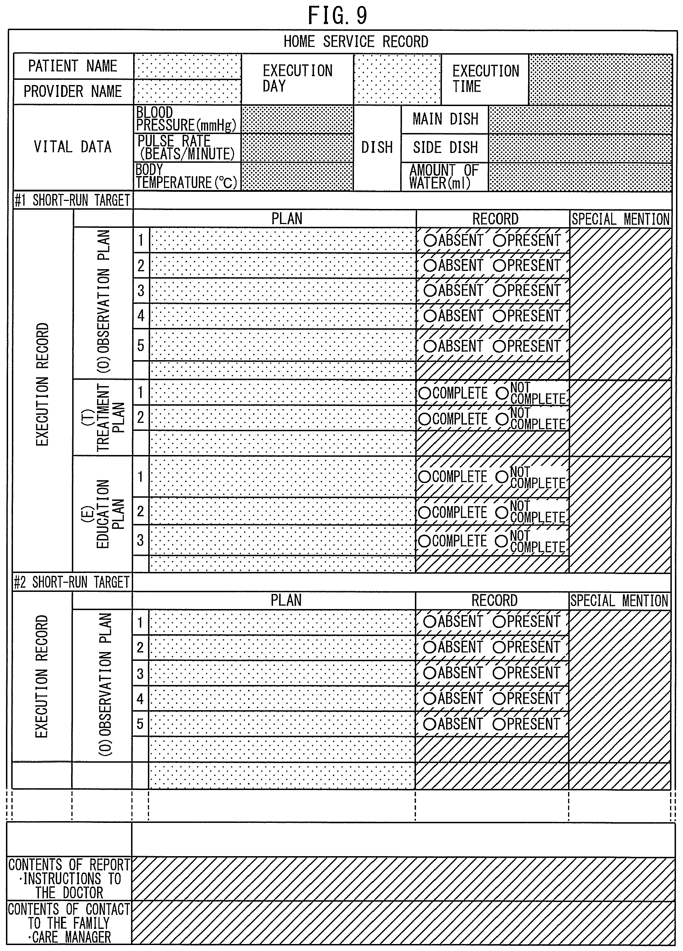

FIG. 9 is a diagram illustrating an example in which the arrangement information on the input possible area and/or the display area defined by a form creator is applied to the scanned form image information 61 in FIG. 8. The arrangement information is defined by the coordinate information or the like on each area. In other words, for the scanned form image information 61, vertex coordinate information on a rectangular area specified by the form creator using a mouse or the like is acquired and the acquired vertex coordinate information on the rectangular area is defined as the arrangement information on each area.

For example, the columns (lightly hatched areas) of user name, provider name, execution day, and Plan indicate the arrangement information on the display area defined by a service provider. The columns (densely hatched areas) of blood pressure (mmHg), pulse rate (beats / minute), body temperature (°C), main dish, side dish, amount of water (ml), and execution time indicate the input possible areas with initial value defined by the form creator. The columns (areas with slashes) of Record, Special mention, Contents of report. instructions to the doctor, Contents of contact to the family · care manager indicate the input possible areas without initial value defined by the form creator. Further, "∘absent ∘present" in the column (area with slashes) of Record is defined as "presence/absence record" and "∘completed onot complete" in the column (area with slashes) of Record is defined as "completed/not complete record" or the like.

The registration unit 333 stores the acquired form image information 61 and the defined arrangement information on the input possible area and/or the display area in the form layer information stored in the storage unit on a form that is generated for a home service (step S102).

For example, the registration unit 333 stores the arrangement information on the input possible area and/or the display area in FIG. 9 defined by the form creator and the scanned form image information 61 in the storage unit as in the form layer information management table in FIG. 5A.

In the example in FIG. 5A, for simplification of explanation, only the vertex coordinates of each display area are described, but it may also be possible to describe predetermined attributes, such as the size and the specific color of the area can be described in the form layer information management table in FIG. 5A.

Further, in the example in FIG. 5A, for simplification of explanation, as the arrangement information on the input possible area without initial value of the form layer information management table, only the columns (areas with slashes) of presence/absence record and Special mention of (O) Observation Plan are described among the three execution records (O: Observation Plan, T: Treatment Plan, E: Education Plan), but the arrangement information on the input possible area without initial value of the form layer information management table also stores the following arrangement information or the like of the columns (areas with slashes) of completed/not complete record and Special mention of the reset of the three execution records, i.e., (T) Treatment Plan and (E) Education Plan: (completed/not complete record 1, each vertex coordinate v), (completed/not complete record 2, each vertex coordinate w), (Special mention of (T) Treatment Plan, each vertex coordinate x), (completed/not complete record 3, each vertex coordinate y), (completed/not complete record 4, each vertex coordinate z), (completed/not complete record 5, each vertex coordinate aa), and (Special mention of (E) Education Plan, each vertex coordinate ab).

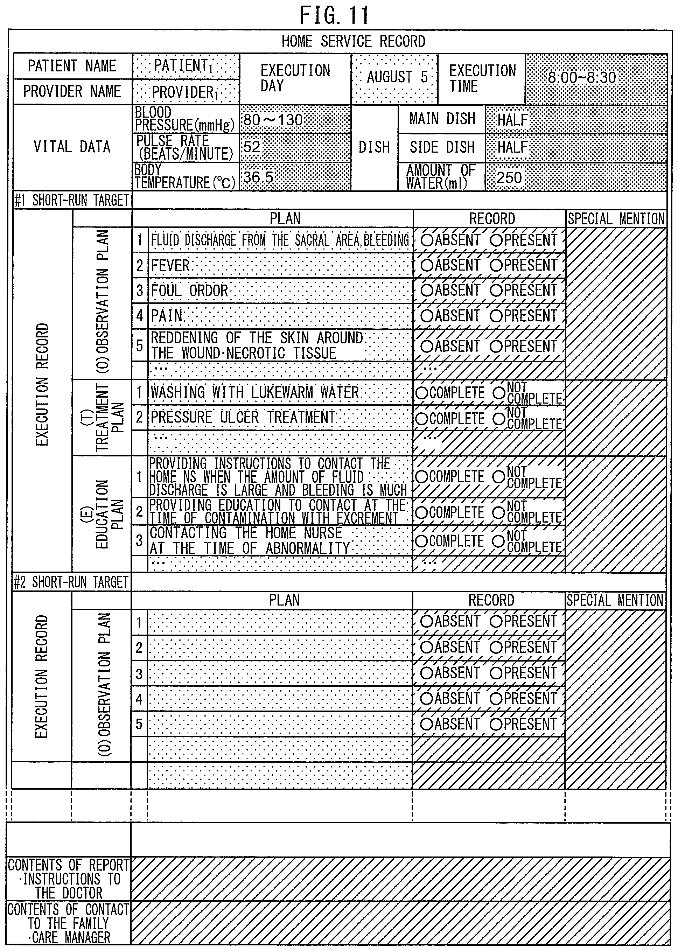

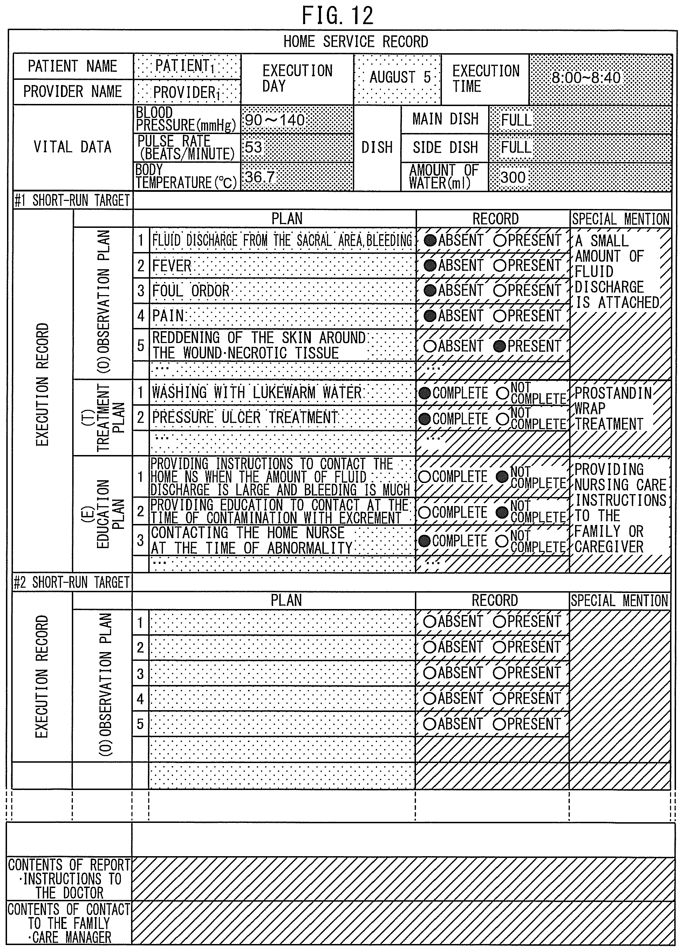

FIG. 10 illustrates an example of an operation sequence by the information management support system 1. With reference to FIG. 11 and FIG. 12, the operation sequence in FIG. 10 is explained.

The operation sequence explained in the following is performed mainly by the device processing unit 25 and the server processing unit 33 in cooperation with each element of the service provider side mobile device 2 and the server 3 based on the program stored in advance in the device storage unit 22 and the server storage unit 32.

The following three pieces of the pre-processing are explained as examples of pre-processing of the operation sequence in FIG. 10. Firstly, the arrangement operation flow of the form layer information by the server is performed. Secondly, the administrator of the organization that provides services sets the ID of a patient, the ID of a service provider, the service provision date and time, the ID of a service action to be provided, and the ID of a form layer of the service provision plan management table, and the ID, the name, and the execution procedure of a service action of the execution procedure management table. Thirdly, the service provider side mobile device 2 of a service provider logs in to the server 3 while providing the home nursing care service on August 5, and transmits the ID of the service provider that is input by the service provider via the operation unit 23 to the server 3.

First, the service provider side mobile device 2 transmits the request for the form information 66 displaying the corresponding written history information 68 of the last time (or an input past date) for a patient along with Patient ID to the server 3 via the device communication unit 21 (step S200). The request for the form information 66 may be any information as long as the information can specify which plan of the service provision plan management table the request for the form information 66 relates to. The past date may include the year. Further, the past date may be a past time in place of a past date.

Next, the form output unit 335 receives the request for the form information 66 from the service provider side mobile device 2 via the server communication unit 31. The form output unit 335 specifies Form layer ID associated with Patient ID by referring to the service provision plan management table and the form layer information management table based on the received request for the form information 66. Then, the form output unit 335 arranges the input possible area and/or the display area in the form image information 61 based on the arrangement information on the form corresponding to the specified Form layer ID (step S201).

For example, it is assumed that the request for the form information 66 is for Patient ID "USER_001". The form output unit 335 refers to the service provision plan management table and the form layer information management table and arranges the input possible area and/or the display area in the form image information 61 based on the arrangement information of which Form layer ID of the form layer information recorded in the record of Patient ID "USER_001" is "LAYER_001, and thus, form image information as illustrated in FIG. 9 is obtained.

When the arrangement information on the input possible area without initial value of the form layer information management table is "presence/absence record", the form output unit 335 arranges a radio button, such as "∘absent opresent". On the other hand, when the arrangement information on the input possible area without initial value of the form layer information management table is "completed/not complete record", the form output unit 335 arranges a radio button, such as "∘completed onot complete".

In the service provision plan management table in FIG. 4A, for simplification of explanation, only "USER_001" is displayed as Patient ID, but information about another patient can be similarly managed in the service provision plan management table.

Next, the extraction unit 334 extracts history information on at least one item of the form relating the home service of the last time (or an input past date) provided to the patient from the written history information 68 by referring to the service provision plan management table and the written history information management table (step S202).

For example, when information for service provision at the last time (at the point in time of August 5) is requested from the service provider side mobile device 2, the extraction unit 334 refers to the service provision plan management table and the written history information management table and specifies the written history information 68 of which Written history ID is "WRITTEN_HISTORY_001" and "WRITTEN_HISTORY_002" from the written history information 68. Then, the extraction unit 334 extracts blood pressure = 80 to 130, pulse rate = 52, body temperature = 36.5, main dish = half, side dish = half, and amount of water = 250, respectively, which are the last time (at the point in time of August 5) items and their values of <August 1>.

Next, the extraction unit 334 refers to the service provision plan management table and the execution procedure management table and specifies Action ID of the execution procedure management table based on Action ID recorded in the service provision plan management table of Patient ID. The extraction unit 334 extracts the execution plan information indicating a plan of performing a home service of at least one item of the form that is generated for the home service (step S203).

For example, the extraction unit 334 refers to the service provision plan management table and the execution procedure management table and extracts, as the execution procedure, "1. fluid discharge from the sacral area, bleeding, 2. fever, 3. foul odor, 4, pain, 5. reddening of the skin around the wound · necrotic tissue", "1. washing with lukewarm water, 2. pressure ulcer treatment, ···", and "1. providing instructions to contact the home NS when the amount of fluid discharge and bleeding is excessive, 2. providing education to contact at the time of contamination with excrement, 3. contacting the home nurse at the time of abnormality", respectively, since Action IDs of the service for Patient ID "USER_001" are "ACTION_001", "ACTION_002", and "ACTION_012". The home NS refers to the home nurse station.

Further, the extraction unit 334 refers to the service provision plan management table and extracts Service date and time of this time (8/5, 8:00 to 8:30). The extraction unit 334 extracts user name and provider name by a well-known method.

Next, the form output unit 335 outputs, along with the form image information 61, the input possible area and/or the display area displaying the extracted history information on at least one item of the form that is generated for the home service, and the input possible area and/or the display area displaying the executed execution plan information indicating a plan of performing a home service of at least one item of the form that is generated for the home service as the form information 66 (step S204).

For example, the form output unit 335 refers to the input possible area with initial value of the form layer information management table and displays blood pressure = 80 to 130, pulse rate = 52, body temperature = 36.5, main dish = half, side dish = half, and amount of water = 250 extracted by the extraction unit 334 at step S202 in each item of the input possible area arranged by the form output unit 335 at step S201.

The form output unit 335 refers to the arrangement information on the display area of the form layer information management table and displays "1. fluid discharge from the sacral area, bleeding, 2. fever, 3. foul odor, 4, pain, 5. reddening of the skin around the wound · necrotic tissue", "1. washing with lukewarm water, 2. pressure ulcer treatment ···", and "1. providing instructions to contact the home NS when the amount of fluid discharge and bleeding is excessive, 2. providing education to contact at the time of contamination with excrement, 3. contacting the home nurse at the time of abnormality" extracted by the extraction unit 334 at step S203 in each item of the display area arranged by the form output unit 335 at step S201.

The form output unit 335 refers to the arrangement information on the input possible area and the display area of the form layer information management table and also displays the extracted information (Service date and time "8/5, 8:00 to 8:30", user name, and provider name) other than the information extracted by the extraction unit 334 at steps S202 and 203 in each item of the input possible area and the display area arranged by the form output unit 335 at step S201.

Then, the form output unit 335 outputs the input possible area and/or the display area displaying each item as the form information 66 along with the form image information 61.

Next, the form output unit 335 transmits the output form information 66 to the service provider side mobile device 2 via the server communication unit 31 (step S205). The browsing execution unit 251 outputs the form information 66 received from the server 3 to the display unit 24 via the device communication unit 21. The service provider browses the form information 66 output to the display unit 24 and inputs written information or the like into the input possible area via the operation unit 23.

FIG. 11 is a diagram illustrating an example of the form information 66 in which the last time written history information 68 on August 1 and the information indicating the plan that is performed on August 5 are output by the display unit 24 of the service provider side mobile device 2 when performing the home service on August 5.

The columns (lightly hatched areas) of user name, provider name, execution day, and Plan of #1 Short-run target indicate the display areas in which information that cannot be modified is displayed. Thus, a service provider cannot input information into or modify information in the display area in which information that cannot be modified is displayed. When #2 Short-run target is planned, in the column (lightly hatched area) of Plan of #2 Short-run target also, information that cannot be modified is displayed.

The columns (densely hatched areas) of blood pressure (mmHg), pulse rate (beats / minute), body temperature (°C), main dish, side dish, amount of water (ml), and execution time indicate the input possible areas with initial value in which the written history information 68 is displayed as an initial value at the time of output of the form information 66. Thus, even if the service provider of this time is different from the service provider of the last time, the service provider of this time can quickly recognize a change in the condition of his/her health, such as the vital sign, between the patient of the last time or a past date and the patient of this time. Further, the service provider can fill in the form based on or by referring to the written information input the last time or in the past, which is displayed in the input possible area, and therefore the service provider can reduce the work load in order to fill in the form.

The columns (areas with slashes) of Record, Special mention, Contents of report · instructions to the doctor, and Contents of contact to the family · care manager indicate the input possible area without initial value. Thus, the service provider who performs a home service can input the execution record into the input possible area via the operation unit 23 while browsing / checking each plan displayed as the execution plan information. Further, the service provider can check the plan and the execution record in the same form, and therefore the service provider can reduce the work load.

By outputting the form information 66 in FIG. 11 to the display unit 24 of the service provider side device, the arrangement of a variety of areas of the conventional paper form becomes the same as that of the form obtained by scanning the paper form. Thus, a service provider may browse and fill in the form without having an uncomfortable feeling due to differences from the paper form, and the time taken for training in filling in the form may be very short.

FIG. 12 is a diagram illustrating an example of the form information 66 in FIG. 11 in which the last time written history information 68 on August 1 and the execution plan information indicating the plan that is performed on August 5 are output by the display unit 24 of the service provider side mobile device 2, and into which a service provider has input the execution record of the execution of the execution plan information on August 5, the vital data, and the information on dishes via the operation unit 23 of the service provider side mobile device 2.
a blood pressure gauge, a pulse rate meter, or a clinical thermometer, not illustrated can measure the vital data (blood pressure, pulse rate, body temperature, etc.) of a patient, and the vital data of the patient can be transmitted to the service provider side mobile device, and the service provider side mobile device receives the vital data of the patient.

The device processing unit 25 transmits the written information input into the input possible area by the service provider via the operation unit 23 to the server 3 via the device communication unit 21 (step S206).

The registration unit 333 receives the written information input into the input possible area via the server communication unit 31. The registration unit 333 refers to the service provision plan management table, the form history information management table, and the written history information management table, and stores the received input written information as the history information of the form history information 67 and as the history information of the written history information 68 corresponding thereto, respectively (step S207). One of the features of the present invention is that the written information input into the input possible area is stored in the state of being associated with the two formats of the form history information 67 and the written history information 68 as described above.

For example, the registration unit 333 registers the written information, such as blood pressure = 90 to 140, pulse rate = 53, body temperature = 36.7, main dish = full, side dish = full, amount of water = 300, and execution time 8:00 to 8:40, which is input into the input possible area with initial value in FIG. 12 in the written history information 68 of the written history information management table along with the date.

Thus, the extraction unit 334 can extract the written information input the last time or in the past from the written history information 68, not from the form history information 67, and to narrow down the range of the extraction target by removing the form image information 61 from the range of the extraction target. Thus, the extraction unit 334 can quickly extract the written information input the last time or in the past.

Further, the extraction unit 334 extracts an item of the written history information 68 also from another form (home service report, home service monitoring, etc.), not from the home service record, and the form output unit 335 causes the written history information 68 on the extracted item to be output in the input possible area and/or an area in the vicinity of the input possible area along with the form image information 61 on the other form. Thus, a service provider can refer to the item once stored and the value of the item from any form and to make use of the value of the same item again without the need to input the value again. The written information also can be used when performing an information analysis or collecting statistics.

The registration unit 333 stores the written information input into the input possible area in FIG. 12, such as blood pressure = 90 to 140, pulse rate = 53, body temperature = 36.7, main dish = full, side dish = full, amount of water = 300, execution time = 8:00 to 8:40, presence/absence record 1 = absent, presence/absence record 2 = absent, presence/absence record 3 = absent, presence/absence record 4 = absent, presence/absence record 5 = present, Special mention of (O) Observation Plan = a small amount of fluid discharge is attached, completed/not complete record 1 = completed, completed/not complete record 2 = completed, Special mention of (T) Treatment Plan = Prostandin wrap treatment, completed/not complete record 3 = not complete, completed/not complete record 4 = not complete, completed/not complete record 5 = completed, and Special mention of (E) Education Plan = providing nursing care instructions to the family or caregiver in the display information in the input possible area of the form history information management table.

The registration unit 333 stores the written information corresponding to each of the columns of user name = user₁, provider name = provider₁, execution day = August 5, and Plan of Execution record, which is displayed in the display area in FIG. 12, in the display information in the display area of the form history information management table. A service provider can use the form history information 67 as a report with no change.

The registration unit 333 stores "WRITTEN_HISTORY_001", "WRITTEN_HISTORY_002", ··· storing the written history information 68 in Written history ID of the service provision plan management table. The registration unit 333 stores "SHEET_HISTORY_131 storing the form history information 67 in Form history ID of the service provision plan management table. Then, the registration unit 333 changes Situation of the corresponding service provision of the service provision plan management table from "not complete" to "completed".

As explained above, when a service provider company provides a service, the information management support system 1 can reduce the work load for a service provider and to improve service provision efficiency.

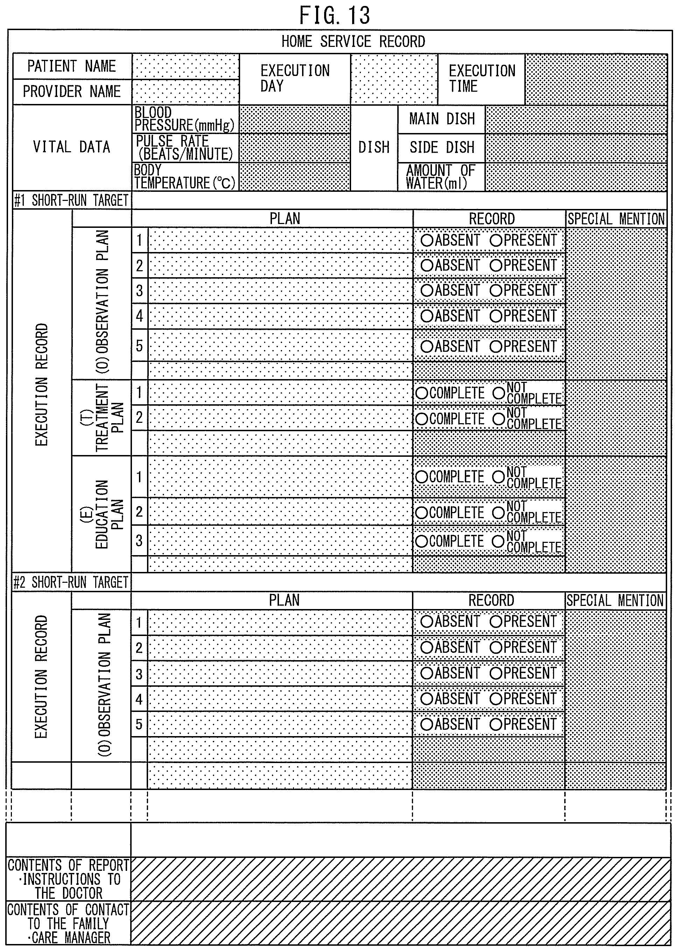

The present invention is not limited to the present embodiment. For example, in the present embodiment, the columns (areas with slashes) of Record and Special mention are defined as the input possible areas without initial value as illustrated in FIG. 9. However, as illustrated in FIG. 13, the columns (densely hatched areas) of Record and Special mention may be defined as input possible areas with initial value. A service provider can quickly recognize a change in Execution record between the patient of the last time or a past date and the patient of this time. Further, a service provider can fill in a form based on or by referring to the written information input the last time or in the past, which is displayed in the input possible area, and therefore the service provider can reduce the work load in order to fill in a form.

Furthermore, in the present embodiment, the form output unit 335 outputs the input possible area displaying the extracted written history information 68 of the last time (or a past date) as the form information 66 along with the form image information 61. However, when outputting the form of this time (e.g., August 5), the form output unit 335 can output, along with the form image information 61, a pulled-down area displaying the written history information 68 corresponding to a predetermined number of times (e.g., three times of the last time (August 1), the second last time (July 28), and the third last time (July 25)) including the last time written history information 68 in the vicinity of the input possible area as the form information 66.

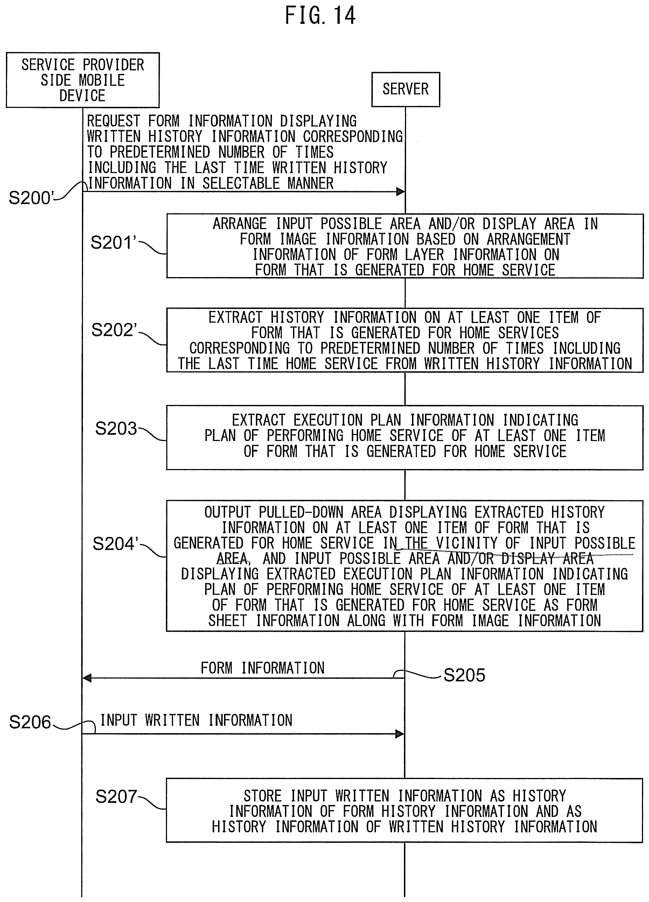

FIG. 14 is a diagram illustrating another example of the operation sequence by the information management support system 1 to output, along with the form image information 61, a pulled-down area displaying the written history information 68 corresponding to a predetermined number of times (e.g., three times of the last time (August 1), the second last time (July 28), and the third last time (July 25)) including the last time written history information 68 in the vicinity of the input possible area as the form information 66.

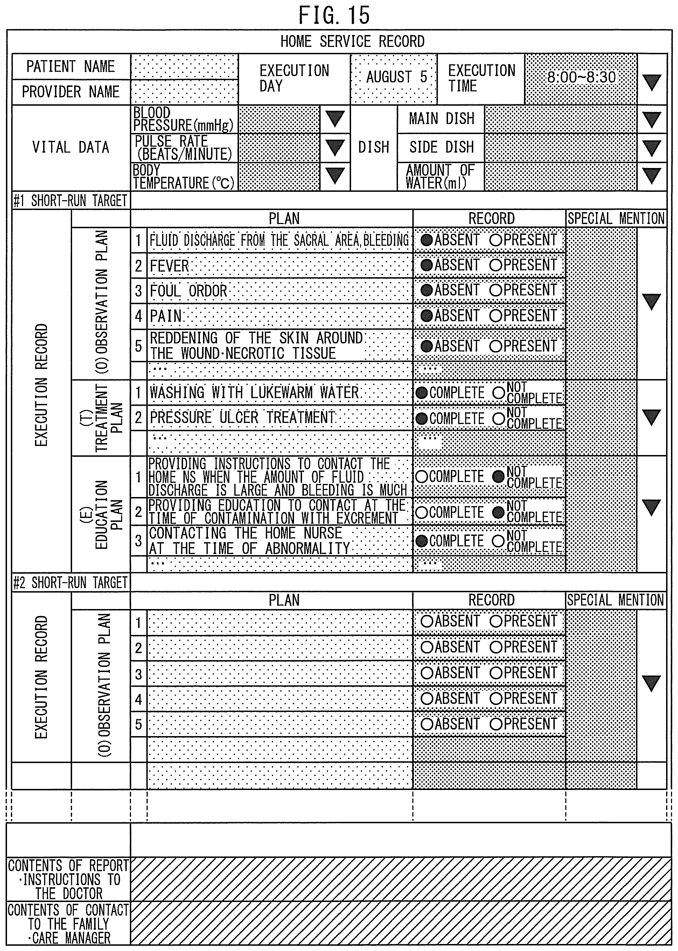

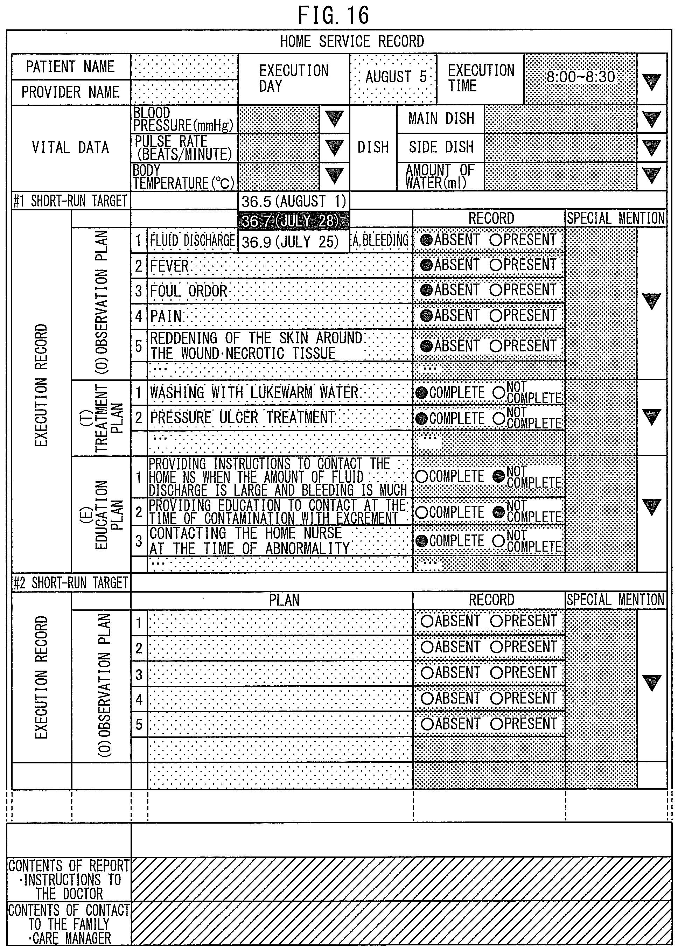

In the operation sequence in FIG. 14, steps S203 and 205 to 207 are performed as in the operation sequence in FIG. 10, but in the operation sequence in FIG. 14, steps S200', S201', S202', and S204' different from steps S200, S201, S202, and S204, respectively, in the operation sequence in FIG. 10 are performed. In the following, with reference to FIGs. 15 and 16, only steps S200', S201', S202', and S204' in the operation sequence in FIG. 14 are explained.

The service provider side mobile device 2 transmits the request for the form information 66 displaying the written history information 68 corresponding to a predetermined number of times including the last time written history information 68 in a selectable manner to the server 3 via the device communication unit 21 (step S200').

The form output unit 335 receives the request for the form information 66 from the service provider side mobile device 2 via the server communication device 31. The form output unit 335 refers to the service provision plan management table and the form layer information management table based on the received request for the form information 66 and specifies Form layer ID associated with Patient ID. Then, the form output unit 335 arranges the input possible area and/or the display area in the form image information 61 based on the arrangement information on the form corresponding to the specified Form layer ID (step S201').

It is explained that the form output unit 335 arranges "presence/absence record" and/or "completed/not complete record". The form output unit 335 can refer to the written history information management table, to acquire a pair of the item checked the last time and its value from the written history information 68 on "presence/absence record" and/or "completed/not complete record", and to arrange "presence/absence record" and/or "completed/not complete record" in the state where the value of the item checked the last time is checked in "presence/absence record" and/or "completed/not complete record". The form output unit 335 can arrange "presence/absence record" and/or "completed/not complete record" as the input possible area without initial value in place of the state of being checked.

The extraction unit 334 extracts history information on at least one item of the form that is generated for the home services corresponding to a predetermined number of times including the last home service from the written history information 68 (step S202').

The form output unit 335 outputs, along with the form image information 61, the pulled-down area displaying the extracted history information on at least one item of the form that is generated for the home service in the vicinity of the input possible area, and the input possible area and/or the display area displaying the extracted execution plan information indicating the plan of performing the home service of at least one item of the form that is generated for the home service as the form information 66 (step S204').

FIG. 15 illustrates an example of the form information 66 in which the pulled-down area displaying the written history information 68 corresponding to a predetermined number of times (e.g., three times of the last time (August 1), the second last time (July 28), and the third last time (July 25)) including the last time written history information 68 in the vicinity of the input possible area is output along with the form image information 61.

The pulled-down area (densely hatched area displaying □ and ▼) is an area in which the written history information 68 is displayed in an area pulled down in the vicinity of the input possible area in a selectable manner and in which the written history information 68 selected by a service provider via the operation unit 23 is input into the input possible area. In other words, the pulled-down area services as both the input possible area and the display area in which the written history information 68 is displayed in a selectable manner in an area pulled down in the vicinity of the input possible area. Further, after selecting the written history information 68 in the pulled-down area in the vicinity of the input possible area, a service provider can modify the selected written history information 68 in the input possible area.

In the pulled-down area of the column of execution time in FIG. 15, the information ("8:00 to 8:30") indicting the execution plan of this time (August 5) extracted by referring to the service provision plan management table is displayed as an initial value. By selecting the pulled-down area of the column of execution time, a service provider can select and input the execution times corresponding to a predetermined number of times (e.g., three times of the last time (August 1), the second last time (July 28), and the third last time (July 25)) including the last time execution time from the written history information 68.

No initial value is displayed in the pulled-down area of the column other than execution time, since it is difficult to determine which initial value of the written history information 68 should be displayed as an initial value among the written history information 68 corresponding to a predetermined number of times (e.g., three times of the last time (August 1), the second last time (July 28), and the third last time (July 25)). The written history information 68 of the last time (August 1) may display in all the pulled-down areas of the columns other than execution time as initial values.

FIG. 16 illustrates an example in which a service provider selects an input possible area (column of body temperature (°C)) and selectably displays the written history information 68 in the pulled-down area in the vicinity of the input possible area (column of body temperature (°C)).

When a service provider selects "36.7 (July 28)" in the pulled-down area in the vicinity of the input possible area of the column of body temperature (°C) in FIG. 16, "36.7" is input into the input possible area of the column of body temperature (°C). The service provider fills in a form by checking or modifying "36.7" input into the input possible area.

Thus, the service provider can select the written history information 68 that is used this time via the operation unit 23 from the written history information 68 of the same item corresponding to a predetermined number of times including the last time written history information 68, and therefore the number of alternatives for input can increased. Further, the amount of data of the written history information 68 corresponding to a predetermined number of times including the last time written history information 68 in the input possible area is far smaller than the amount of data of the entire form information 66 including the form images corresponding to a predetermined number of times including the last time form image. Thus, the extraction unit 334 can quickly extract the written history information 68 corresponding to a predetermined number of times including the last time written history information 68 in the input possible area and further, i the service provider side mobile device 2 can quickly output the form information 66 to the display unit 24.

As to the input possible area other than the column of body temperature (°C) in FIG. 16, the service provider sequentially continues to fill in the form in the pulled-down area in the vicinity of the input possible area while selecting, via the operation unit 23, any piece of the written history information 68 corresponding to a predetermined number of times including the last time written history information 68.

Then, the registration unit 333 stores the pair of "presence/absence record" and/or "completed/not complete record", and the value of the item in the written history information 68 of the written history information management table along with the date.

In the present embodiment, the information input into the form information 66 via the operation unit 23, which the form output unit 335 causes the display unit 24 of the service provider side mobile device 2 to output, is not checked. However, the form output unit 335 can output the form information 66 to which a function to check input information is attached. The function to check input information is implemented by a software program being attached to the form information 66.

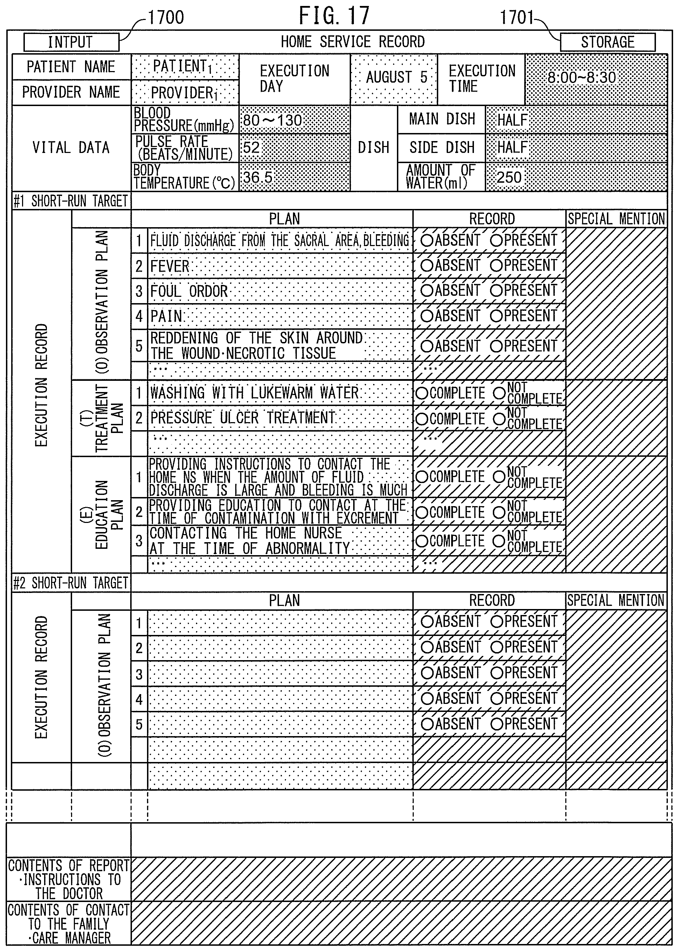

FIG. 17 is a diagram illustrating an example of the form information 66 to which the function to check input information is attached.

The columns of Home service record of the form information 66 output to the display unit 24 of the service provider side mobile device 2 are the same as those in FIG. 11, but two buttons, i.e., an Input button 1700 at the top-left of the form information 66 and a Save button 1701 on the top-right are further added. The Input button 1700 and the Save button 1701 are buttons that do not exist in a paper form and are displayed as buttons that do not belong to any layer in FIG. 6A.

When the Input button 1700 is pressed down (clicked) by a service provider via the operation unit 23 while the execution plan information including history information is being displayed on the display unit 24 after step S205 in FIG. 10 described above, the last time history information that has been hitherto displayed disappears from the input possible area and the input possible area enters a state of awaiting an input.

On the other hand, when the Save button 1701 is pressed down (clicked) by a service provider via the operation unit 23 after information is input into the input possible area by the service provider via the operation unit 23, the check function attached to the form information 66 checks the item in the input possible area. Then, the check function attached to the form information 66 extracts an item that is not filled in despite that the filling-in of the item is indispensable and displays the column of the item in a conspicuous color, such as red, or displays a message of "Unfilled".

The check function attached to the form information 66 may check an item other than the item the filling-in of which is indispensable described above. For example, the check function attached to the form information 66 can extract an item that is not filled in with a figure despite that the filling-in with a figure of the item is indispensable and to display the column of the item in a conspicuous color, such as red, or to display a message of "Filling-in with a figure is indispensable". Further, the check function attached to the form information 66 can extract an item that is not filled in with a date or a time despite that the filling-in with a date or a time of the item is indispensable and to display the column of the item in a conspicuous color, such as red, or to display a message of "Filling-in with a date is indispensable" or "Filling-in with a time is indispensable".

Further, for example, the check function attached to the form information 66 may check the input vital data by comparing the input vital data with the last time vital data of the written history information 68. In this case, if the input vital data has changed from the last time vital data of the written history information 68 by a predetermined value or a predetermined ratio or more, the check function attached to the form information 66 changes the color of the input possible area of the input vital data that has changed by a predetermined value or a predetermine ratio or more, or causes the display unit 24 to display a warning message. A service provider can quickly recognize a change in the vital data by a predetermined value or a predetermined ratio or more between the patient of the last time or a past date and the patient of this time.

By further attaching the check function for the input operation into the displayed information, in addition to the last time history information display, a service provider may perform the series of processing, such as processing to refer to the past history and processing to record the action performed based on the history that is referred to, with a feeling close to that when working with conventional paper. As explained above, the information management support system 1 may reduce the work load for a service provider and to improve service provision efficiency in addition to making an attempt to reduce the training time of the service provider when a service provider company provides a service.

Further, the computer program for causing a computer to implement each function of the device processing unit 25 and the server processing unit 33 may be provided in the form of being stored in a computer readable storage medium, such as a semiconductor storage medium, a magnetic storage medium, and an optical storage medium, and may be installed in the server storage unit 32 from the storage medium by using a publicly known setup program or the like.

It should be understood that a person skilled in the art can make various changes, substitutions, and modifications to the present invention without deviating from the sprit and scope of the present invention.

### Explanation of letters or numerals

- 1: information management support system
- 2: service provider side mobile device
- 21: device communication unit
- 22: device storage unit
- 23: operation unit
- 24: display unit
- 25: device processing unit
- 251: browsing execution unit
- 3: server
- 31: server communication unit
- 32: server storage unit
- 33: server processing unit
- 331: form image information acquisition unit
- 332: form arrangement information acquisition unit
- 333: registration unit
- 334: extraction unit
- 335: form output unit
- 4: base station
- 5: mobile communication network
- 6: gateway
- 61: form image information
- 62: layer information on display area
- 63: layer information on input possible area with initial value
- 64: layer information on input possible area without initial value
- 65: form layer information
- 66: form information
- 67: form history information
- 68: written history information
- 7: Internet

## Claims

1. A control method of a server in an information management support system comprising a service provider side mobile device relating to a service provider who provides a service and a server capable of communicating with the service provider side mobile device, the server comprising a storage unit configured to store: form history information including form image information on a form that is generated for a service provided by a service provider and history information input in correspondence to at least one item of the form image information; and written history information indicating written information of history information input in correspondence to at least one item of a form that is generated for the service for each patient, wherein
the server performs:
a step of receiving written information input for a form that is generated for a service provided by a service provider from the service provider side mobile device;
a step of storing the received input written information as history information of the form history information stored in the storage unit and as history information of the written history information stored in the storage unit;
a step of extracting history information on at least one item of a form that is generated for a service from the written history information stored in the storage unit; and
a step of causing the service provider side mobile device to output, along with form image information, an input possible area and/or an area in the vicinity of the input possible area displaying the extracted history information on at least one item of a form that is generated for a service so that a service provider can browse the history information.

2. The control method of a server according to claim 1, wherein
in the storing step, an execution date and/or an execution time is stored as history information of the form history information stored in the storage unit and the written history information stored in the storage unit,
a step of receiving a past date and/or time input by a service provider from the service provider side mobile device is further performed, and
in the extracting step, history information on at least one item of a form that is generated for a service, which is specified by the received past date and/or time, is extracted from the written history information stored in the storage unit.

3. The control method of a server according to claim 1, wherein
the storage unit further stores execution plan information indicating a plan of performing a service of at least one item of a form that is generated for a service provided by a service provider,
execution plan information stored in the storage unit indicating a plan of performing a service of at least one item of a form that is generated for a service is extracted in the extracting step, and
a display area displaying the extracted execution plan information indicating a plan of performing a service of at least one item of a form that is generated for a service and an input possible area into which an execution record is input by a service provider who performs the service are output along with form image information in the outputting step.

4. The control method of a server according to claim 1, wherein
the storage unit further stores form layer information including form image information on a form that is generated for a service and arrangement information on an input possible area and/or a display area,
a step of acquiring form image information obtained by scanning a form that is generated for a service, a step of acquiring arrangement information defining an input possible area and/or a display area in the acquired form image information, and a step of storing the acquired form image information and the acquired arrangement information on an input possible area and/or a display area in the form layer information stored in the storage unit on a form that is generated for a service are further performed, and
an input possible area and/or a display area is arranged in form image information based on arrangement information of the form layer information stored in the storage unit on a form that is generated for a service and further, the extracted history information on at least one item of a form that is generated for a service is displayed in an input possible area and/or a display area arranged in the form image information so that a service provider can browse the history information in the outputting step.

5. A server in an information management support system comprising a service provider side mobile device relating to a service provider who provides a service and a server capable of communicating with the service provider side mobile device, the server comprising:
a storage unit configured to store: form history information including form image information on a form that is generated for a service provided by a service provider and history information input in correspondence to at least one item of the form image information; and written history information indicating written information of history information input in correspondence to at least one item of a form that is generated for the service for each patient:
a communication unit configured to receive written information input for a form that is generated for a service provided by a service provider from the service provider side mobile device;
a registration unit configured to store the received input written information as history information of the form history information stored in the storage unit and as history information of the written history information;
an extraction unit configured to extract history information on at least one item of a form that is generated for a service from the written history information stored in the storage unit; and
a form output unit configured to cause the service provider side mobile device to output, along with form image information, an input possible area and/or an area in the vicinity of the input possible area displaying the extracted history information on at least one item of a form that is generated for a service so that a service provider can browse the history information.

6. A control program of a server in an information management support system comprising a service provider side mobile device relating to a service provider who provides a service and a server capable of communicating with the service provider side mobile device, the server comprising a storage unit configured to store: form history information including form image information on a form that is generated for a service provided by a service provider and history information input in correspondence to at least one item of the form image information; and written history information indicating written information of history information input in correspondence to at least one item of a form that is generated for the service for each patient, wherein
the control program causes the server to perform:
a step of receiving written information input for a form that is generated for a service provided by a service provider from the service provider side mobile device;
a step of storing the received input written information as history information of the form history information stored in the storage unit and as history information of the written history information stored in the storage unit;
a step of extracting history information on at least one item of a form that is generated for a service from the written history information stored in the storage unit; and
a step of causing the service provider side mobile device to output, along with form image information, an input possible area and/or an area in the vicinity of the input possible area displaying the extracted history information on at least one item of a form that is generated for a service so that a service provider can browse the history information.
